# EUROPEAN PATENT SPECIFICATION

(11) **EP 2 543 027 B1**
(45) Date of publication and mention of the grant of the patent: **24.08.2016**
(21) Application number: 10841751.0
(22) Date of filing: 30.12.2010
(51) Int. Cl.: A61M 16/06, A61B 5/103, A61B 5/00, A61B 5/11, A61F 5/56

(54) **DEVICE FOR MONITORING SLEEP POSITION**
Vorrichtung zur Überwachung der Schlafposition
Appareil de surveillance de la position de sommeil

(30) Priority: 31.12.2009 US 335067 P
(43) Date of publication of application: 09.01.2013
(73) Proprietor: NIGHTBALANCE B.V., 2629 JD Delft (NL)
(72) Inventor: de VRIES, Nicolaas, 1077 XB Amsterdam (NL); BOUCHER, Ryan P., San Francisco, CA 94107 (US); DOELLING, Eric N., Sunnyvale, CA 94087 (US)
(74) Representative: EP&C
(86) International application number: PCT/US2010/062576
(87) International publication number: WO 2011/082349

(56) References cited:
- JP-A- 2007 202 939
- US-A- 5 081 447
- US-A- 5 914 660
- US-A1- 2003 144 596
- US-A1- 2007 277 836
- US-A1- 2008 094 226
- US-A1- 2009 251 285
- US-A1- 2009 281 433
- US-B1- 7 106 190

## Description

### Field of the Invention

The invention generally relates to a system for helping individuals experiencing sleep apnea, snoring, or other forms of sleep obstructive breathing achieve deep, restorative sleep.

US 2008/0094226 describes a system to prevent sudden infant death syndrome. The system detects that the child wearing a disposable absorbent article is in the predetermined position which may be a potentially life-threatening position. The position sensor is arranged on the disposable absorbent article.

US 5081447 discloses an apparatus for influencing a person to sleep on his side and not on his back. A gravity actuated sensor apparatus is attached to a sleeping person by a strap worn on the body or on the head. The apparatus includes an alarm connected to the sensor apparatus so that the alarm is actuated in response to the sensor apparatus detecting an undesired body position.

JP 2007-202939 discloses a neck band including biological-information detecting part which contacts a test subjects neck and detects biological information.

US 2009/0281433, on which the preamble of claim 1 is based, discloses a system for pulmonary monitoring and treatment including an oral appliance and one or more sensors mounted to the oral appliance to capture intra-oral data. The sensors can be temperature sensors, flow velocity sensors, acoustic sensors, heart rate sensors, optical sensors, arterial tone sensors, oxygen sensors, EEG sensors, EKG sensors, PH sensors, or snoring sound sensors. The system can provide therapy to a patient in that the system can wake the patient in the event that a sleep apnea condition, a snoring condition, a pulmonary condition or a bruxing condition is detected. Waking of the patient can be done by sound, vibration or electrical energy.

### Background of the Invention

Approximately fifty-six percent (56%) of sleep apnea sufferers are position dependent. Position dependent OSA has been defined when an individual experiences at least two times as many apneic events when sleeping in one of the four principal sleeping positions: left side, right side, prone (on the stomach), or supine (on the back). Snoring is often position dependent and is reduced when a patient changes its position.

### Summary of the Invention

The invention relates in particular to a system according to claim 1.

The System provides a position sensing component that carries a gravity sensor sized and configured to provide a position dependent output indicative of the relative sleep position of the individual. According to the invention, the position sensing component is integrated into a therapeutic oral appliance sized and configured to be worn within an oral cavity of the individual. The position sensing component is worn by the individual during sleep.

The system compares the position dependent output with one or more benchmark conditions that correlate to a desired sleep position. The system generate an alarm output comprising an audible sound and/or tactile vibration when the individual is not in a desired sleep position, to thereby influence or alter the individual's sleep position to return the individual to a sleep position that correlates to a desired sleep position.

In one embodiment, the system senses the duration of a particular position dependent output before generating an alarm output to prevent a false alarm.

### Brief Description of the Drawings

Fig. 1 is a perspective view of a therapeutic system for monitoring sleep position, the system including first, second, and third components that serve complementary sensing, monitoring, and corrective functions, respectively.
Figs. 2A and 2B are views of gravity-position sensor that serves a sensing function in the system shown in Fig. 1, being integrated into a therapeutic oral appliance, which is worn in the oral cavity of an individual during sleep for the treatment for obstructive sleep apnea.
Fig. 3 is a diagrammatic view of the complementary sensing, monitoring, and corrective functions performed by the system shown in Fig. 1, including set, preprogrammed rules that establish a desired sleep position conducive to deep, restorative sleep.

### Description of the Preferred Embodiments

Although the disclosure hereof is detailed and exact to enable those skilled in the art to practice the invention, the physical embodiments herein disclosed merely exemplify the invention, which may be embodied in other specific structure. While the preferred embodiment has been described, the details may be changed without departing from the invention, which is defined by the claims.

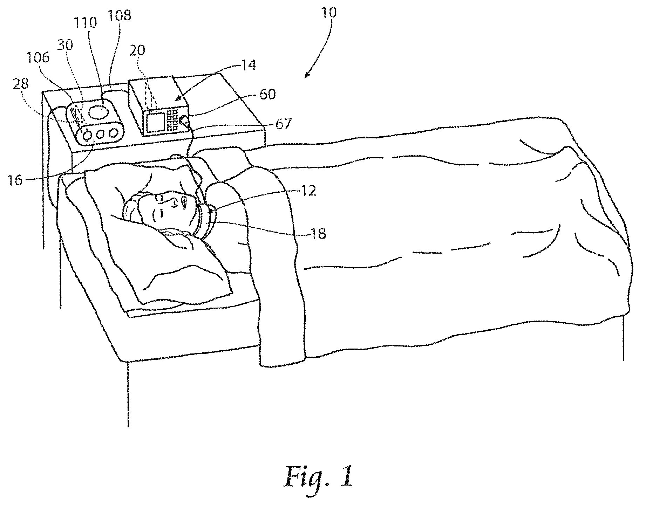

Fig. 1 depicts a representative therapeutic system 10 which is sleep position sensitive. The system includes the first, second, and third components 12, 14, and 16. Functionally, the three components 12, 14, and 16 desirably serve to teach or prompt the individual to assume a desired sleeping position, which is defined as the sleep position or positions most conducive to deep, restorative sleep for the individual.

The first component 12 serves a position sensing function. In the embodiment shown in Fig. 1 the first component 12 includes a strap that is sized and configured to be worn about the neck, which does not form part of the invention.

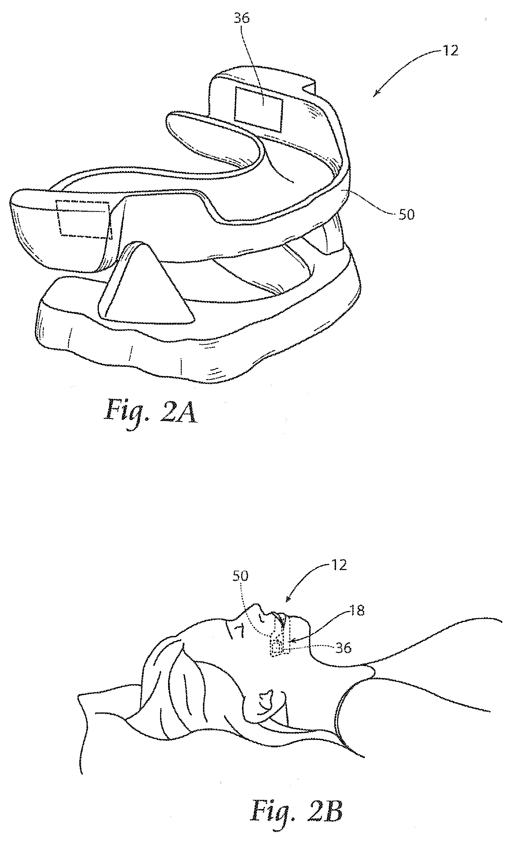

In the embodiment shown in Figs. 2A and 2B, which is according to the invention, the first component 12 is incorporated into a conventional therapeutic oral appliance 50. The term "therapeutic oral appliance" means an oral appliance that fits over the upper and lower teeth and is sized and configured, to hold the tongue and/or push the lower jaw forward and serve as an alternative to CPAP therapy for the treatment of obstructive sleep apnea. The therapeutic oral appliance 50 is configured for temporary placement into and removal from the oral cavity (as Fig. 2B shows). Thus, the therapeutic oral appliance 50 may be used only during sleep and removed upon awakening. Removal of the therapeutic oral appliance 50 during waking hours prevents any interference with swallowing, speech, or other routine activities.

In this arrangement, the first component 12 includes, as the sensing element 18, a gravity sensor 36 integrated into the therapeutic oral appliance 50. As before described, the gravity sensor 36 can be of conventional form. For example, it can take the form of a mechanical or electromechanical instrument based on "the bubble in a liquid" concept, like a Mercury gravity switch; or a gravity type potentiometer; or a capacitive gravity sensor; or other forms of miniaturized integrated circuit technologies, such as micro-switches; or forms of micro-electromechanical systems (MEMS).

When the therapeutic oral appliance 50 is worn in the oral cavity (as Fig. 2B shows), the position dependent output of the gravity sensor 36 is indicative of the relative position of the individual's head on the sleeping surface. The position dependent output indicates whether the individual is resting on the left side of the head, right side of the head, back of the head, or facedown.

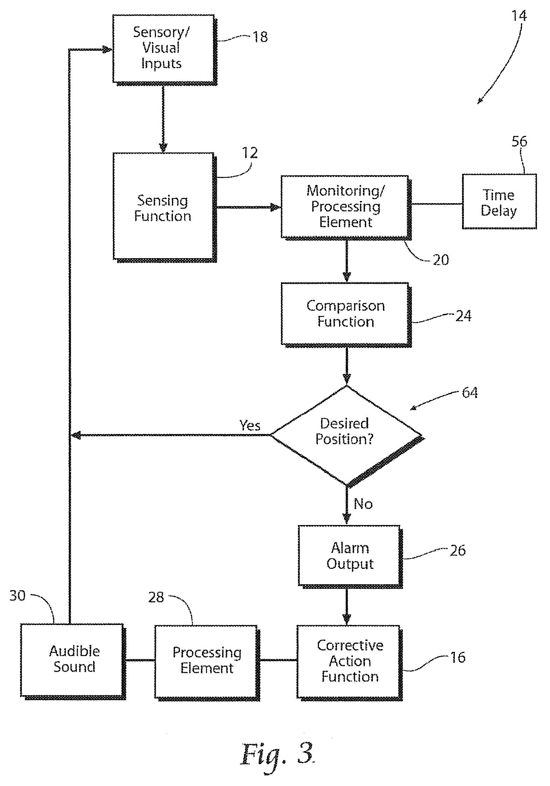

In the illustrated embodiment (see Figs. 1 and3), the second component 14 serves a monitoring function. The second component 14 receives the position-indicative output of the first component 12. As Figs. 1 and 3 show, the second component 14 includes a processing element 20 that continuously or periodically registers the position- indicative output. As shown in Fig.3, the processing element 20 includes a comparison function 24, which compares the registered position-indicative output with one or more benchmark or threshold conditions that correlate to a desired sleep position. When a correlation is lacking (meaning that the individual is not in the desired sleep position), the processing element generates an alarm output.

Desirably (as Fig. 3 diagrammatically shows), the processing element 20 includes a time-delay function 56 that senses the duration of a particular position- indicative output before registering it. In this way, transient changes of position are not registered and processed by comparison function 24. False alarms are thereby eliminated or reduced. The duration of the time delay can be incorporated into the pre-programmed rules and, desirably, be adjusted based upon false alarms experienced.

The form and fit of the second component 14 can vary. For example, as seen in Fig. 1, the second component 14 can be incorporated into a compact housing 60 sized and configured to be placed bed-side. The housing 60 desirably houses the processing element 20, which can comprise a microprocessor implemented on an integrated circuit board.

Communication between the first and second components can be accomplished by linking the two components with a transmission cable 67 (shown in solid lines in Fig. 1) . Alternatively, a wireless communication channel can be established between the two components, e.g., using an infrared transceiver.

The second component 14 can be battery powered, either by use of a standard industry-standard primary battery or an industry-standard rechargeable battery.

In one embodiment (as diagrammatically shown in Fig.3), the processing element 20 of the second component 14 can include set, pre-programmed rules 64 that establish one or more sleep positions as the desired sleep position. The desired sleep position or positions can be ascertained by diagnosis of individual data or by analysis statistical patient population samples, or both.

For example, a left side and right side sleep position can be pre-programmed in the processing element as being desired. These sleep positions thereby become the benchmark conditions.

By continuously or periodically registering the position-indicative output of the first component 12, and by comparing the position-indicative output to the benchmark conditions or rules 64, the processing element 20 of the second component 14 either ascertains a correlation exists (i.e., the individual is resting in a desired sleep position) or ascertains that a correlation is lacking (i.e., the individual is resting on their back or front). As Fig. 3 shows, when a correlation is lacking, the processing element generates the alarm output 26. The alarm output 26 is transmitted to the third component 16 for inducing a change in the sleeping position.

The third component 16 is coupled to the second component. The third component 16 includes a processing element 28 that responds to the respective alarm output (based upon sleep position) and generates a corrective output.

The third component 16 also includes a corrective action element 30 that responds to the alarm output to influence or alter the individual's sleep position, to a return the individual to a sleep position that correlates to the desired benchmark conditions. Return to the benchmark conditions results in the return to deep, restorative sleep. Return to the desired benchmark conditions interrupts the alarm input.

As shown in Fig. 1, in any one of the systems described, the third component 16 can comprise a housing 106 sized and configured to be placed bed-side separate from the second component 14. The housing 106 desirably houses the processing element 28 of the third component 16, which can comprise a microprocessor implemented on an integrated circuit board. Communication between the second and third components 14 and 16 can be accomplished by linking the two components with a transmission cable 108 (shown in Fig. 1). Alternatively, a wireless communication channel can be established between the two components, e.g., using an infrared transceiver. Alternatively, the second and third component 16 can be incorporated or fully integrated incorporated into a single housing. The first, second, and third components 12, 14, and 16 can all be integrated into the therapeutic oral appliance 50 itself, to provide a fully self- contained system 10.

As shown in Fig. 1, the corrective action element 30 is coupled to a speaker 110 through which an audible sound is generated to awake the individual. The individual, now aroused, is forced to change his sleeping position to silence the sound.

Alternatively, or in combination, the corrective action element 30 can comprise an electrical buzzer or vibrator carried on the therapeutic oral appliance 50. The buzzer provides a tactile vibration sensation to awake the individual. The individual, now aroused, is forced to change his sleeping position to silence the vibration.

Alternatively, or in combination, the correct action element may adjust the therapeutic oral appliance 50 to extend the jaw forward to generate a larger airway.

Operation of the corrective action element 30 disturbs the individual in an auditory way sufficient to arouse the individual, thereby teaching the individual to alter their sleep position or posture and thereby return to a sleep state more conducive to deep, restorative sleep. The above-described embodiments of this invention are merely descriptive of its principles and are not to be limited. The scope of this invention instead shall be determined from the scope of the following claims, including their equivalents.

## Claims

1. A system comprising an oral appliance (50) sized and configured to be worn in an oral cavity of an individual and a sensor (18) carried by the oral appliance (50),
**characterized in that**
the system comprises:
a position sensing component (12) that is integrated in the oral appliance, wherein the oral appliance is a therapeutic oral appliance (50) that fits over the upper and lower teeth of the individual and is sized and configured to hold the tongue and/or push the lower jaw forward, and wherein the sensor (18) is a gravity sensor (36) sized and configured to provide a position dependent output indicative of the relative sleep position of the individual, a monitoring component (14) communicating with the gravity sensor (36) to receive the position dependent output, the monitoring component (14) including a processing element (20) that compares the position dependent output with one or more benchmark conditions that correlate to a desired sleep position, the processing element generating an alarm output (26) when the individual is not in a desired sleep position, and
a corrective action component (16) communicating with the monitoring component (14) including a corrective action element (30) that, in response to the alarm output (26), generates an audible sound and/or tactile vibration to influence or alter the individual's sleep position to return the individual to a sleep position that correlates to a desired sleep position.

2. System according to claim 1, wherein the gravity sensor (36) comprises a mechanical or electromechanical a bubble in a liquid switch; or a gravity type potentiometer; or a capacitive gravity sensor; or a miniaturized integrated circuit; or a micro- electromechanical device.

3. System according to claim 1, wherein the processing element (20) of the monitoring component (14) includes a time-delay function (56) that senses the duration of a particular position dependent output before generating an alarm output to prevent a false alarm.

4. System according to any one of the preceding claims, wherein the position sensing component (12), the monitoring component (14) and the corrective action component (16) are all integrated into the therapeutic oral appliance (50).

5. System according to claim 4, wherein the corrective action component (16) comprises an electrical buzzer or vibrator carried on the therapeutic oral appliance (50).

6. System according to claim 4 or 5, wherein the corrective action element is adapted to adjust the therapeutic oral appliance (50) to extend the jaw forward to generate a larger airway.

## Patentansprüche

1. Ein System, das eine Mundvorrichtung (50), die so bemessen und konfiguriert ist, dass sie in einer Mundhöhle eines Individuums angeordnet werden kann, und einen an der Mundvorrichtung (50) befestigten Sensor (18) aufweist,
**dadurch gekennzeichnet, dass**
das System umfasst:
- ein Positionserfassungsteil (12), das in der Mundvorrichtung (50) integriert ist, wobei die Mundvorrichtung eine therapeutische Mundvorrichtung (50) ist, die über die oberen und unteren Zähne des Individuums passt und so bemessen und konfiguriert ist, die Zunge zu halten und / oder den Unterkiefer nach vorn zu schieben, und wobei der Sensor (18) ein Schwerkraftfühler (36) ist, der bemessen und konfiguriert ist, ein lageabhängiges Ausgangssignal, das die relative Schlafposition des Individuums angibt, bereitzustellen, eine Überwachungskomponente (14), die mit dem Schwerkraftfühler (36) kommuniziert, um das lageabhängige Ausgangssignal zu erhalten, die Überwachungskomponente (14) umfasst ein Verarbeitungselement (20), das das lageabhängige Ausgangssignal mit einer oder mehreren Referenzbedingungen vergleicht, die mit einer gewünschten Schlafposition korrelieren, das Verarbeitungselement generiert eine Alarmausgabe (26), wenn sich das Individuum nicht in einer gewünschten Schlafposition befindet, und
- ein Korrekturmaßnahme-Bauteil (16), das mit der Überwachungskomponente (14) kommuniziert und ein Korrekturmaßnahme-Element (30) aufweist, das in Reaktion auf die Alarmausgabe (26) ein hörbares Geräusch und / oder eine taktile Vibration erzeugt, um die Schlafposition des Individuums zu beeinflussen oder zu verändern und das Individuum in eine Schlafposition zurückzubringen, die mit einer gewünschten Schlafposition korreliert.

2. System nach Anspruch 1, wobei der Schwerkraftfühler (36) einen mechanischen oder elektromechanischen eine Blase in einer Flüssigkeit aufweisende Schalter, oder einen kapazitiven Gravitationssensor, oder einen miniaturisierten integrierten Schaltkreis, oder eine mikroelektromechanische Vorrichtung aufweist.

3. System nach Anspruch 1, wobei das Verarbeitungselement (20) der Überwachungskomponente (14) eine Zeitverzögerungsfunktion (56) aufweist, die die Dauer eines bestimmten lageabhängigen Ausgangssignals erfasst, bevor eine Alarmausgabe erfolgt, um einen Fehlalarm zu verhindern.

4. System nach einem der vorhergehenden Ansprüche, wobei das Positionserfassungsteil (12), die Überwachungskomponente (14) und das Korrekturmaßnahme-Bauteil (16) alle in der therapeutischen Mundvorrichtung (50) integriert sind.

5. System nach Anspruch 4, wobei das Korrekturmaßnahme-Bauteil (16) einen elektrischen Summer oder Vibrator umfasst, der an der therapeutischen Mundvorrichtung (50) angeordnet ist.

6. System nach Anspruch 4 oder 5, wobei das Korrekturmaßnahme-Bauteil ausgelegt ist, die therapeutische Mundvorrichtung (50) so einzustellen, dass der Unterkiefer nach vorne bewegt wird, um einen größeren Luftweg zu erzeugen.

## Revendications

1. Système comprenant un appareil buccal (50) dimensionné et configuré pour être porté dans une cavité buccale d'une personne et un capteur (18) porté par l'appareil buccal (50),
**caractérisé en ce que**
le système comprend :
un composant de détection de position (12) qui est intégré dans l'appareil buccal, dans lequel l'appareil buccal est un appareil buccal thérapeutique (50) qui se place sur les dents supérieures et inférieures de la personne et qui est dimensionné et configuré pour maintenir la langue et/ou pousser la mâchoire inférieure vers l'avant, et dans lequel le capteur (18) est un capteur de gravité (36) dimensionné et configuré pour produire un signal de sortie, dépendant de la position, qui indique la position relative de la personne pendant le sommeil, un composant de surveillance (14) communiquant avec le capteur de gravité (36) pour recevoir le signal de sortie dépendant de la position, le composant de surveillance (14) incluant un élément de traitement (20) qui compare le signal de sortie, dépendant de la position, avec une ou plusieurs conditions de référence qui sont en corrélation avec une position souhaitée pendant le sommeil, l'élément de traitement générant en sortie un signal d'alarme (26) lorsque la personne ne se trouve pas dans la position souhaitée pendant le sommeil, et
un composant d'action corrective (16) communiquant avec le composant de surveillance (14) et incluant un élément d'action corrective (30) qui, en réponse au signal d'alarme (26), génère un son audible et/ou une vibration tactile destinés à influencer ou modifier la position de la personne pendant le sommeil afin de ramener la personne dans une position de sommeil qui est en corrélation avec une position de sommeil souhaitée.

2. Système selon la revendication 1, dans lequel le capteur de gravité (36) comprend un commutateur mécanique ou électromécanique du type bulle dans liquide ; ou un potentiomètre du type à gravité ; ou un capteur de gravité capacitif ; ou un circuit intégré miniaturisé ; ou un dispositif micro-électromécanique.

3. Système selon la revendication 1, dans lequel l'élément de traitement (20) du composant de surveillance (14) inclut une fonction de temporisation (56) qui détecte la durée d'un signal de sortie particulier, dépendant de la position, avant de générer en sortie un signal d'alarme pour empêcher une fausse alarme.

4. Système selon l'une quelconque des revendications précédentes, dans lequel le composant de détection de position (12), le composant de surveillance (14) et l'élément d'action corrective (16) sont tous intégrés dans l'appareil buccal thérapeutique (50).

5. Système selon la revendication 4, dans lequel le composant d'action corrective (16) comprend une sonnerie ou un vibrateur électrique porté sur l'appareil buccal thérapeutique (50).

6. Système selon la revendication 4 ou 5, dans lequel l'élément d'action corrective est adapté pour régler l'appareil buccal thérapeutique (50) pour que celui-ci étende la mâchoire vers l'avant et agrandisse les voies aériennes.
